# EUROPEAN PATENT SPECIFICATION

(11) **EP 2 826 470 B1**
(45) Date of publication and mention of the grant of the patent: **06.09.2017**
(21) Application number: 14177607.0
(22) Date of filing: 18.07.2014
(51) Int. Cl.: A61K 9/16, A61K 9/48, A61K 31/197, A61K 31/714

(54) **Pharmaceutical combinations of pregabalin**
Pharmazeutische Kombinationen von Pregabalin
Combinaisons pharmaceutiques de prégabaline

(30) Priority: 19.07.2013 TR 201308785
(43) Date of publication of application: 21.01.2015
(73) Proprietor: Arven Ilac Sanayi ve Ticaret A.S., Istinye, Istanbul 34460 (TR)
(72) Inventor: Türkyilmaz, Ali, 34460 Istanbul (TR); Pehlivan Akalin, Nur, 34460 Istanbul (TR); Demir, Vildan, 34460 Istanbul (TR); Eceoglu, Melike, 34460 Istanbul (TR)
(74) Representative: Sevinç, Erkan

(56) References cited:
- EP-A1- 2 343 055
- WO-A1-2013/100873
- WO-A1-2013/100874
- WO-A1-2013/109199
- CA-A1- 2 859 487
- NARMADA P ET AL: "RP-HPLC method development and validation for the determination of methylcobalamin and pregabalin in combined capsule dosage form", INTERNATIONAL JOURNAL OF RESEARCH IN PHARMACEUTICAL SCIENCES,, vol. 4, no. 1, 5 May 2013 (2013-05-05), pages 25-29, XP009177546, ISSN: 0975-7538

## Description

### Field of Invention

The present invention relates to a pharmaceutical formulation, comprising pregabalin or a pharmaceutically acceptable salt of pregabalin and vitamin B12 or an active form of vitamin B12 in the form of a capsule.

### Background of Invention

Epilepsy is a disorder of brain electrical activity that results in recurrent seizures. Conventional treatment of epilepsy consists primarily of anticonvulsant medications. Pregabalin is an analog of gamma-aminobutyric acid (GABA). Its chemical designation is (S)-3-(aminomethyl)-5-methyl hexanoic acid, with the chemical structure illustrated below with Formula 1.

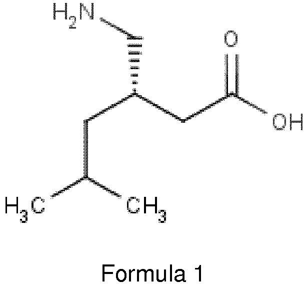

It is known that pregabalin binds to the alpha2-delta (A₂D) receptors of an auxiliary subunit of voltage-sensitive calcium channels in the central nervous system, thereby replacing [3H]-gabapentin. It also reduces the release of glutamate, norepinephrine, substance P, and other neurotransmitters which carries pain signals. It is used for the treatment of epilepsy, simple or complex partial convulsion, either accompanied or not by secondary generalized convulsions, and of neuropathic pain. Molecule and indications are disclosed in US 5 563 175 and various applications can be found in relation to pregabalin formulations. It is generally provided in the form of conventional capsule formulations and posology requires such formulations to be administered twice or thrice daily.

Although pregabalin controls and reduces the frequency of seizures, some patients may not show sufficient improvement due to its side effects. It is important that people with epilepsy follow a nutritious, well balanced diet together with drug therapy. Good nutritional habits and a healthy lifestyle are important in obtaining optimal seizure control. A number of dietary modifications and nutritional supplements are recommended for patients with epilepsy treatment to avoid dietary deficiencies, to reduce the nerve damage and other side effects of epileptic drugs.

One of the most common side effects of pregabalin is vitamin B deficiency which causes nerve damage and anemia. In human body, all the members of the vitamin B family play crucial roles in promoting and insuring nerve health. Thiamine (B1) and biotin (B7) promote healthy nerves. Riboflavin (B2) aids in nerve insulation. Niacin (B3) assists nervous system function, while pyridoxine (B6) helps the body use and absorbs niacin and B12. A lack of B6 may cause carpal tunnel syndrome, painful neuropathy in your hands. Folic acid (B9) deficiency causes neuropathic leg and foot pain. Among all of them, the most important is vitamin B12 which helps nerves function and avoid damages.

Vitamin B12 is water-soluble and has the most complex chemical structure of all the vitamins with its corrin structure and a cobalt ion at its center (formula 2). It is decomposed by light and UV irradiation. Its chemical name is α-(5,6-dimethylbenzimidazolyl)cobamidcyanide and it is called as cyanocobalamin, cobalamin, cyomin, crystamine, berubigen, coobalamed, cobamin, cobione, cynobal, dobetin or cobavite. Its active forms are called as hydroxocobalamin, adenosylcobalamin or methylcobalain. Vitamin B12 is necessary for the production of red blood cells and plays an important role in maintaining the health of nerve cells and in the formation of genetic material. Deficiency results in anemia and nervous system damages which cause loss of balance, numbness and weakness and trigger the seizures of epilepsy patients.

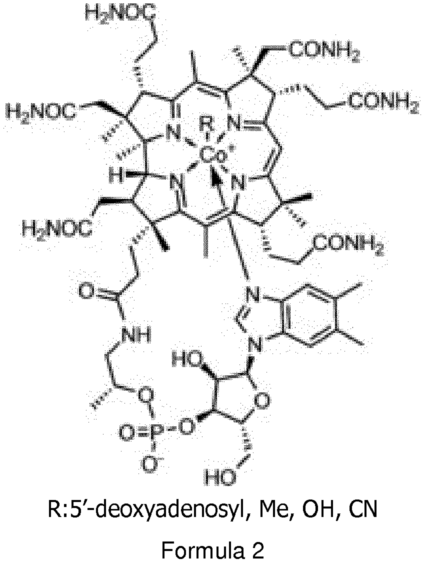

For all these reasons, to have more effective epilepsy treatment, it is recommended to use pregabalin together with the supplementation of vitamin B12 with or without vitamin B₆ and folate. The combination of pregabalin and vitamin B12 has been described in the prior art. CA 2 859 487 A1 discloses a combination of an antiepileptic agent and B-complex vitamins, particularly pregabalin and vitamin B12. The compositions of CA 2 859 487 A1 offers a synergistic combination which provides fewer adverse effects and long term efficacy.

The use of pregabalin along with vitamin B12 is also described in a literature of "International Journal of Research in Pharmaceutical science"; vol.4, no.1, 5 May 2013, pages 25-29.

Pharmaceutical formulations of pregabalin and methods for the preparation of these formulations are disclosed in WO 2013/100873 A1. WO 2013/100873 A1 further disclose the use of vitamin B12 as a second active agent in pregabalin containing formulations. The method directed to the method of granulation; wherein granules comprising pregabalin are sieved and pulverized such that they have an average particle size in the range of 300-2500 µm. Considering this, combinations of pregabalin and vitamin B12 in a suitable pharmaceutical dosage formulation is needed for epilepsy patients. In prior art, capsule dosage form of pregabalin has been disclosed in EP2343055A1 in which pregabalin stability problem has been overcome , but any capsule formulation for combination of pregabalin and vitamin B12 has not been disclosed. Vitamin B12 has also stability problem due to UV irradiation sensitivity and therefore, capsule formulation for the combination of pregabalin and vitamin B12 is also needed.

In addition to be a solution for stability problem, capsule usage has also advantages depending on the patient's needs. It is easier than tablets for people to swallow because of their shape, odor and taste masking. In contrast to tablets, capsules cannot be cut into pieces by patients, so misuse or undesired dose of drugs can be prevented. Moreover, it reduces the gastrointestinal irritation and allows faster dissolution and absorption of active agents.

On the other side, there are many challenges while combining two or more molecules in a capsule such as (a) the physicochemical compatibility between the different active ingredients and/or between the active ingredients and the excipients used; and (b) the therapeutical compatibility between the two active ingredients regarding their pharmacokinetic and/or pharmaceutical properties in order that the posology of the combined formulation allows to obtain safe and efficient plasma levels of both pharmacological agents; and (c) lower incidence of side effects.

It is also known that obtaining an adequate content uniformity is an important issue especially in pharmaceutical formulations comprising more than one active ingredient. Because it is difficult to homogenize the active ingredients, especially if they have low doses and high bulk density in final dosage form, difficulties may occur when filling into the capsules during the process and this may cause inadequate content uniformity of the final dosage forms. As a result of this, undesired effects can be occurred during the therapy.

Thus, there is a need in the art for capsule formulations for combination of pregabalin or a pharmaceutically acceptable salt thereof and vitamin B12 or an active form of vitamin B12 provides improved anticonvulsant effects with reduced seizures and nerve damages.

### Description of Invention

The present invention relates to a pharmaceutical formulation, comprising pregabalin or a pharmaceutically acceptable salt of pregabalin and vitamin B12 or an active form of vitamin B12 and a disintegrant; wherein said formulation is in the form of a capsule and the vitamin B12 is in the range of 0.15 to 10.0%, preferably 0.25 to 5% by weight of total formulation; and said disintegrant is sodium starch glycolate.

"Active forms" of vitamins are defined as activated vitamins which don't require a conversion by the liver to be absorbed. Liver convert the inactive forms of vitamins quickly and while they are absorbed, some part of them may also be excreted from the body before the absorption. On the other side, in the active forms of vitamins, conversion is already carried out to achieve immediate and high absorption of vitamins.

According to one object of the present invention is to provide a pharmaceutical formulation wherein pregabalin or a pharmaceutically acceptable salt is present in an amount of about 20 - 90 % by weight of total formulation, preferably it is about 25 - 75 % by weight of total formulation.

The main object of the present invention is to obtain a combination of pregabalin or a pharmaceutically acceptable salt of pregabalin and vitamin B12 or an active form of vitamin B12 providing improved anticonvulsant effects with reduced seizures and nerve damages.

One object of the present invention is, a pharmaceutical formulation comprises the vitamin B12 in the range of 0.15 to 10.0%, preferably 0.25 to 5% by weight of total formulation and the ratio of pregabalin to vitamin B12 is in the range of 20 to 300 (w/w) and preferably 25 to 150 (w/w).

Another object of the present invention is to ensure the patient compliance and stability of each active agent by providing a capsule formulation comprising the combination of pregabalin and vitamin B12 optionally with vitamin B₆ and B₉. Said capsule is opaque in order to protect vitamin B12 from the sunlight and UV irradiation.

Another object of the present invention is to obtain a pharmaceutical formulation to overcome content uniformity problems by providing homogeneity, low bulk density and flowability.

According to another object, said formulation comprises one or more pharmaceutically acceptable excipients that are selected from the group comprising disintegrants, fillers, binders, diluents, lubricants, glidants, sweeteners and flavouring agents.

We have surprisingly found that, the selection of excipients and their proportions have importance to obtain an adequate content uniformity. Thus, suitable excipients have been investigated to obtain adequate homogeneity and content uniformity.

The disintegrant is sodium starch glycolate.

The amount of sodium starch glycolate is in the range of 1 to 70 %, preferably 5 to 30 % by weight of total formulation and the ratio of pregabalin to sodium starch glycolate is in the range of 1 to 30 (w/w), preferably 1 to 15 (w/w).

While the effectiveness of many disintegrants is changing by the presence of hydrophobic excipients such as lubricants or glidants, the disintegrant efficiency of sodium starch glycolate remains intact. In this invention, to achieve desired homogeneity of pregabalin and vitamin B12 formulation, sodium starch glycolate has been used as a disintegrant which is not previously used in prior art. Moreover, when it has been used in this formulation as a disintegrant, it has been observed that it increased the flowability of powder.

In another object, for powder formulations it is very difficult to achieve acceptable content uniformity. Especially, at low doses it is hard to disintegrate low amount (25 mg) of vitamin B12 homogeneously. To overcome this problem, vitamin B12 with sodium starch glycolate has been granulated via wet granulation technique before preparing the total mixture.

In one object, suitable filler are selected from the group comprising microcrystalline cellulose, lactose, sucrose, glucose, sorbitol, inorganic salts, dibasic calcium phosphate dehydrate, anhydrous dibasic calcium phosphate, or mixtures thereof; preferably the filler is anhydrous dibasic calcium phosphate.

Anhydrous dibasic calcium phosphate is nonhygroscopic and stable at room temperature. Two main particle-size grades of anhydrous dibasic calcium phosphate are used in the pharmaceutical industry. The milled material is typically used in wet-granulated, roller-compacted or slugged formulations. The "unmilled" or "coarse-grade" material is typically used in direct-compression formulations.

In this invention, during the development study of this formulation, inventors faced with a flowabilty problem because vitamin B12 with pregabalin and anhydrous dibasic calcium phosphate increases the bulk density of total formulation (especially at low doses) and shows the flowability problem which makes capsule filling difficult. During the process, while filling the capsules, the powder mixture should have an optimum flowability and bulk density to ensure the content uniformity. Therefore, it is found that using anhydrous dibasic calcium phosphate coarse-grade powder as filler due to its high flowability property decreases the bulk density of the total formulation and improves the total content uniformity.

The amount of anhydrous dibasic calcium phosphate is in the range of 10 to 90 %, preferably 50 to 70 % by weight of total formulation and the ratio of pregabalin to anhydrous dibasic calcium phosphate is in the range of 0.2 to 5 (w/w), preferably 0.4 to 4 (w/w).

The glidants selected from the group comprising colloidal silicon dioxide, colloidal anhydrous silica, talc, aluminium silicate or mixtures thereof; preferably colloidal silicon dioxide.

The amount of colloidal silicon dioxide is in the range of 0.1- 5 %, preferably 0.70 - 2 % by weight of total formulation and the ratio of pregabalin to colloidal silicon dioxide is in the range of 10 - 100 (w/w), preferably 30 - 100 (w/w).

Furthermore, it has surprisingly found that particle size of excipients affects the flowability of total formulation. Sodium starch glycolate with finer particle size and/or dibasic calcium phosphate with finer particle size have decreased the bulk density of total powder. When bulk density was decreased, flowability increases and capsules are filled easily by providing content uniformity.

In this invention, particle sizes of sodium starch glycolate and/or dibasic calcium phosphate have been measured by *Malvern Mastersizer 2000 laser diffraction particle size analyzer* by dry dispersion method and d₉₀ value of these excipients have been identified. "d₉₀" or "d(0.9)" is defined as the size value corresponding to volume of the particles at 90%, which represents the size of particles below which 90% of the substance lies.

Sodium starch glycolate has a particle size diameter range with a d₉₀ value between 80 to 10 µm, preferably 80 to 50 µm and d₉₀ value of coarse-grade powder anhydrous dibasic calcium phosphate is in the range of 120 to 30 µm, preferably 120 to 60 µm.

In this present invention, to achieve capsules comprising pregabalin and B12 these formulations have been designed, comprising the following:
a. 20.0 - 90.0 % by weight of pregabalin
b. 0.15 - 10.0 % by weight of vitamin B12
c. 10 - 90.0 % by weight of anhydrous dibasic calcium phosphate
d. 1.0 - 70 % by weight of sodium starch glycolate
e. 0.1 - 5.0 % by weight of colloidal silicon dioxide
or,
a. 25.0 - 75.0 % by weight of pregabalin
b. 0.25 - 5.0 % by weight of vitamin B12
c. 50.0 - 70.0 % by weight of anhydrous dibasic calcium phosphate
d. 5.0 - 30 % by weight of sodium starch glycolate
e. 0.70 - 2 % by weight of colloidal silicon dioxide

The process for preparing the pharmaceutical formulation according to this invention comprising the steps of;
- granulation vitamin B12 and sodium starch glycolate with ethanol
- drying granules
- sieving the granules and mixing with pregabalin, colloidal silicon dioxide and anhydrous dibasic calcium phosphate.
- capsule filling with the powder mixture.

### Example 1:

| **ingredient** | **amount %** |
|---|---|
| pregabalin | 25.0 |
| Vitamin B12 | 1.0 |
| anhydrous dibasic calcium phosphate (white coarse powder) | 53.25 |
| sodium starch glycolate | 20.0 |
| colloidal silicon dioxide | 0.75 |

### Example 2:

| **ingredient** | **amount %** |
|---|---|
| pregabalin | 75.0 |
| Vitamin B12 | 0.5 |
| anhydrous dibasic calcium phosphate (white coarse powder) | 18.75 |
| sodium starch glycolate | 5.0 |
| colloidal silicon dioxide | 0.75 |

The process of the formulations is carried out as follows: vitamin B12 and sodium starch glycolate are granulated with ethanol. Granules are dried at 45 ºC. Dried granules sieved and mixed with pregabalin, colloidal silicon dioxide and anhydrous dibasic calcium phosphate coarse-grade powder, then filled into capsules.

## Claims

1. A pharmaceutical formulation, comprising pregabalin or a pharmaceutically acceptable salt of pregabalin and vitamin B12 or an active form of vitamin B12, and a disintegrant wherein said formulation is in the form of a capsule and the vitamin B12 is in the range of 0.15 to 10.0%, preferably 0.25 to 5% by weight of total formulation; and said disintegrant is sodium starch glycolate.

2. The pharmaceutical formulation according to claim 1, further comprising pharmaceutically acceptable excipients selected from the group comprising fillers, binders, diluents, lubricants, glidants, sweeteners and flavouring agents.

3. The pharmaceutical formulation according to claim 1, wherein sodium starch glycolate is in the range of 1 - 70 %, preferably 5 - 30 % by weight of total formulation.

4. The pharmaceutical formulation according to any of the preceding claims, wherein the weight ratio of pregabalin to sodium starch glycolate is in the range of 1 to 30 (w/w), preferably 1 to 15 (w/w).

5. The pharmaceutical formulation according to any of the preceding claims, wherein the particle size of sodium starch glycolate is in the range of 80 to 10 µm, preferably 80 to 50 µm.

6. The pharmaceutical formulation according to Claim 2, wherein the fillers are selected from the group comprising anhydrous dibasic calcium phosphate, microcrystalline cellulose, lactose, sucrose, glucose, sorbitol, inorganic salts, dibasic calcium phosphate dehydrate, or mixtures thereof; preferably the filler is anhydrous dibasic calcium phosphate.

7. The pharmaceutical formulation according to claim 6, wherein anhydrous dibasic calcium phosphate is in the range of 10 - 90 %, preferably 50 - 70 % by weight of total formulation.

8. The pharmaceutical formulation according to any of the preceding claims, wherein the weight ratio of pregabalin to anhydrous dibasic calcium phosphate is in the range of 0.2 to 5 (w/w), preferably 0.4 to 4 (w/w).

9. The pharmaceutical formulation according to any of the preceding claims, wherein the particle size of anhydrous dibasic calcium phosphate is in the range of 120 to 30 µm, preferably 120 to 60 µm.

10. The pharmaceutical formulation according to claim 2, wherein said glidant is colloidal silicon dioxide.

11. The pharmaceutical formulation according to claim 10, wherein colloidal silicon dioxide is in the range of 0.1 - 5 %, preferably 0.7 - 2 % by weight of total formulation.

12. The pharmaceutical formulation according to any of the preceding claims, wherein the weight ratio of pregabalin to colloidal silicon dioxide is in the range of 10 - 100 (w/w), preferably 30 - 100 (w/w).

13. The pharmaceutical formulation according to any of the preceding claims, comprises:
a. 20.0 - 90.0 % by weight of pregabalin
b. 0.15 - 10.0 % by weight of vitamin B12
c. 10 - 90.0 % by weight of anhydrous dibasic calcium phosphate
d. 1.0 - 70 % by weight of sodium starch glycolate
e. 0.1 - 5.0 % by weight of colloidal silicon dioxide

14. The pharmaceutical formulation according to any of the preceding claims, comprises:
a. 25.0 - 75.0 % by weight of pregabalin
b. 0.25 - 5.0 % by weight of vitamin B12
c. 50.0 - 70.0 % by weight of anhydrous dibasic calcium phosphate
d. 5.0 - 30 % by weight of sodium starch glycolate
e. 0.70 - 2 % by weight of colloidal silicon dioxide

## Patentansprüche

1. Pharmazeutische Formulierung, umfassend Pregabalin oder ein pharmazeutisch verträgliches Salz von Pregabalin und Vitamin B12 oder eine aktive Form von Vitamin B12 und ein Sprengmittel, wobei die Formulierung in der Form einer Kapsel vorliegt und das Vitamin B12 in dem Bereich von 0,15 bis 10 Gew.-%, vorzugsweise 0,25 bis 5 Gew.-%, der Gesamtformulierung liegt; und das Sprengmittel Natriumstärkeglycolat ist.

2. Pharmazeutische Formulierung gemäß Anspruch 1, ferner umfassend pharmazeutisch verträgliche Hilfsstoffe ausgewählt aus der Gruppe umfassend Füllstoffe, Bindemittel, Verdünnungsmittel, Schmiermittel, Fließregulierungsmittel, Süßstoffe und Aromastoffe.

3. Pharmazeutische Formulierung gemäß Anspruch 1, wobei Natriumstärkeglycolat in dem Bereich von 1-70 Gew.-%, vorzugsweise 5-30 Gew.-%, der Gesamtformulierung liegt.

4. Pharmazeutische Formulierung gemäß einem der vorstehenden Ansprüche, wobei das Gewichtsverhältnis von Pregabalin zu Natriumstärkeglycolat in dem Bereich von 1 bis 30 (w/w), vorzugsweise 1 bis 15 (w/w), liegt.

5. Pharmazeutische Formulierung gemäß einem der vorstehenden Ansprüche, wobei die Partikelgröße von Natriumstärkeglycolat in dem Bereich von 80 bis 10 µm, vorzugsweise 80 bis 50 µm, liegt.

6. Pharmazeutische Formulierung gemäß Anspruch 2, wobei die Füllstoffe ausgewählt sind aus der Gruppe umfassend wasserfreies dibasisches Calciumphosphat, mikrokristalline Cellulose, Lactose, Saccharose, Glucose, Sorbit, anorganische Salze, dibasisches Calciumphosphat Dehydrat oder Gemische davon; vorzugsweise ist der Füllstoff wasserfreies dibasisches Calciumphosphat.

7. Pharmazeutische Formulierung gemäß Anspruch 6, wobei wasserfreies dibasisches Calciumphosphat in dem Bereich von 10-90 Gew.-%, vorzugsweise 50-70 Gew.-%, der Gesamtformulierung liegt.

8. Pharmazeutische Formulierung gemäß einem der vorstehenden Ansprüche, wobei das Gewichtsverhältnis von Pregabalin zu wasserfreiem dibasischem Calciumphosphat in dem Bereich von 0,2 bis 5 (w/w), vorzugsweise 0,4 bis 4 (w/w), liegt.

9. Pharmazeutische Formulierung gemäß einem der vorstehenden Ansprüche, wobei die Partikelgröße von wasserfreiem dibasischem Calciumphosphat in dem Bereich von 120 bis 30 µm, vorzugsweise 120 bis 60 µm, liegt.

10. Pharmazeutische Formulierung gemäß Anspruch 2, wobei das Fließregulierungsmittel kolloidales Siliciumdioxid ist.

11. Pharmazeutische Formulierung gemäß Anspruch 10, wobei kolloidales Siliciumdioxid in dem Bereich von 0,1-5 Gew.-%, vorzugsweise 0,7-2 Gew.-%, der Gesamtformulierung liegt.

12. Pharmazeutische Formulierung gemäß einem der vorstehenden Ansprüche, wobei das Gewichtsverhältnis von Pregabalin zu kolloidalem Siliciumdioxid in dem Bereich von 10-100 (w/w), vorzugsweise 30-100 (w/w), liegt.

13. Pharmazeutische Formulierung gemäß einem der vorstehenden Ansprüche, umfassend:
a. 20,0-90,0 Gew.-% Pregabalin,
b. 0,15-10,0 Gew.-% Vitamin B12,
c. 10-90,0 Gew.-% wasserfreies dibasisches Calciumphosphat,
d. 1,0-70 Gew.-% Natriumstärkeglycolat,
e. 0,1-5,0 Gew.-% kolloidales Siliciumdioxid.

14. Pharmazeutische Formulierung gemäß einem der vorstehenden Ansprüche, umfassend:
a. 25,0-75,0 Gew.-% Pregabalin,
b. 0,25-5,0 Gew.-% Vitamin B12,
c. 50,0-70,0 Gew.-% wasserfreies dibasisches Calciumphosphat,
d. 5,0-30 Gew.-% Natriumstärkeglycolat,
e. 0,70-2 Gew.-% kolloidales Siliciumdioxid.

## Revendications

1. - Formulation pharmaceutique comprenant de la prégabaline ou un sel pharmaceutiquement acceptable de prégabaline et de la vitamine B12 ou une forme active de vitamine B12, et un délitant, ladite formulation étant dans la forme d'une capsule et la vitamine B12 étant dans la plage de 0,15 à 10,0 %, de préférence de 0,25 à 5 % en poids de la formulation totale ; et ledit délitant étant le glycolate d'amidon sodique.

2. - Formulation pharmaceutique selon la revendication 1, comprenant en outre des excipients pharmaceutiquement acceptables choisis dans le groupe comprenant les charges, les liants, les diluants, les lubrifiants, les agents de glissement, les édulcorants et les arômes.

3. - Formulation pharmaceutique selon la revendication 1, dans laquelle le glycolate d'amidon sodique est dans la plage de 1 - 70 %, de préférence de 5 - 30 % en poids de la formulation totale.

4. - Formulation pharmaceutique selon l'une quelconque des revendications précédentes, dans laquelle le rapport en poids de prégabaline au glycolate d'amidon sodique est dans la plage de 1 à 30 (p/p), de préférence de 1 à 15 (p/p).

5. - Formulation pharmaceutique selon l'une quelconque des revendications précédentes, dans laquelle la dimension de particule de glycolate d'amidon sodique est dans la plage de 80 à 10 µm, de préférence de 80 à 50 µm.

6. - Formulation pharmaceutique selon la revendication 2, dans laquelle les charges sont choisies dans le groupe comprenant le phosphate de calcium dibasique anhydre, la cellulose microcristalline, le lactose, le sucrose, le glucose, le sorbitol, les sels inorganiques, le phosphate de calcium dibasique déshydraté, ou leurs mélanges ; de préférence, la charge est le phosphate de calcium dibasique anhydre.

7. - Formulation pharmaceutique selon la revendication 6, dans laquelle le phosphate de calcium dibasique anhydre est dans la plage de 10 - 90 %, de préférence de 50 - 70 % en poids de la formulation totale.

8. - Formulation pharmaceutique selon l'une quelconque des revendications précédentes, dans laquelle le rapport en poids de prégabaline au phosphate de calcium dibasique anhydre est dans la plage de 0,2 à 5 (p/p), de préférence de 0,4 à 4 (p/p).

9. - Formulation pharmaceutique selon l'une quelconque des revendications précédentes, dans laquelle la dimension de particule de phosphate de calcium dibasique anhydre est dans la plage de 120 à 30 µm, de préférence de 120 à 60 µm.

10. - Formulation pharmaceutique selon la revendication 2, dans laquelle ledit agent de glissement est le dioxyde de silicium colloïdal.

11. - Formulation pharmaceutique selon la revendication 10, dans laquelle le dioxyde de silicium colloïdal est dans la plage de 0,1 - 5 %, de préférence de 0,7 - 2 % en poids de la formulation totale.

12. - Formulation pharmaceutique selon l'une quelconque des revendications précédentes, dans laquelle le rapport en poids de prégabaline au dioxyde de silicium colloïdal est dans la plage de 10 - 100 (p/p), de préférence de 30 - 100 (p/p).

13. - Formulation pharmaceutique selon l'une quelconque des revendications précédentes, qui comprend :
a. 20,0 - 90,0 % en poids de prégabaline
b. 0,15 - 10,0 % en poids de vitamine B12
c. 10 - 90,0 % en poids de phosphate de calcium dibasique anhydre
d. 1,0 - 70 % en poids de glycolate d'amidon sodique
e. 0,1 - 5,0 % en poids de dioxyde de silicium colloïdal.

14. - Formulation pharmaceutique selon l'une quelconque des revendications précédentes, qui comprend :
a. 25,0 - 75,0 % en poids de prégabaline
b. 0,25 - 5,0 % en poids de vitamine B12
c. 50,0 - 70,0 % en poids de phosphate de calcium dibasique anhydre
d. 5,0 - 30 % en poids de glycolate d'amidon sodique
e. 0,70 - 2 % en poids de dioxyde de silicium colloïdal.
